# EUROPEAN PATENT SPECIFICATION

(11) **EP 3 842 077 B1**
(45) Date of publication and mention of the grant of the patent: **19.10.2022**
(21) Application number: 20208490.1
(22) Date of filing: 18.11.2020
(51) Int. Cl.: A61L 24/00, A61L 24/04

(54) **EMBOLIZATION PARTICLES AND METHOD FOR PREPARING THE SAME**
EMBOLISATIONSTEILCHEN UND VERFAHREN ZU IHRER HERSTELLUNG
PARTICULES D'EMBOLISATION ET LEUR PROCÉDÉ DE PRÉPARATION

(30) Priority: 23.12.2019 KR 20190172791; 25.08.2020 KR 20200107058
(43) Date of publication of application: 30.06.2021
(73) Proprietor: Plmicromed Co., Ltd., Yangsan-si, Gyeongsangnam-do 50620 (KR)
(72) Inventor: Kim, Gwang Suk, 50594 Gyeongsangnam-do (KR)
(74) Representative: Manitz Finsterwald Patent- und Rechtsanwaltspartnerschaft mbB

(56) References cited:
- JP-A- 2010 083 788
- KR-A- 20170 095 626
- US-A1- 2010 081 790

## Description

### CROSS-REFERENCE TO RELATED APPLICATION

This application claims the benefit under 35 USC 119(a) of Korean Patent Applications Nos. 10-2019-0172791 filed on December 23, 2019, and 10-2020-0107058 filed on August 25, 2020, in the Korean Intellectual Property Office.

### Technical Field

The present disclosure relates to novel embolization particles and a method for preparing the same.

### Background

Embolization or embolotherapy is a substance that treats a tumor by inserting a specific substance into a blood vessel to occlude the blood that delivers oxygen and nutrients to a tumor tissue.

As a general embolic inserting substance must have biocompatibility, hydrophile property, non-toxicity, and biodegrability conditions, microparticles such as chitosan, starch, gelatin, albumin, and sodium alginic acid are mainly studied as embolic materials.

In particular, gelatin is used by applying a product developed as a hemostatic product by using mammal-derived (cows and pigs) gelatin in the 1960s, and a form thereof is irregular because a porous sponge sheet type is cut or mixed. It is difficult for clinicians to expect a desired embolic effect because the gelatin granules are entangled during the microconduit embolization.

The mammal-derived gelatin embolic substance has concerns about transmission of mad cow disease of cattle and infection such as the foot-and-mouth disease of pigs. Religiously and culturally, Hindus who do not want cow-related intake and Muslim who avoid pig-related intake need gelatin that can replace the mammal-derived gelatin.

JP 2010-083788 A discloses an embolization agent obtained by crosslinking gelatin having a molecular weight of 30,000 to 300,000.

### SUMMARY

The present disclosure relates to novel embolization particles and a method for preparing the same.

A first aspect of the present disclosure provides embolization particles according to claim 1.

A second aspect of the present disclosure provides a method for preparing embolization particles according to claim 4.

The embolization particles according to the exemplary embodiments of the present disclosure can have a fast degradation rate, and after a first surgical procedure is performed on a patient, it can be replaced with other blood vessels of the patient several times to perform the surgical procedure depending on patient's conditions. Therefore, the embolization particles according to an example can effectively treat the patient.

The microparticles for embolization according to the exemplary embodiments of the present disclosure are prepared by using polypeptide derived from gelatin. Accordingly, the embolization particles according to the exemplary embodiments of the present disclosure are excellent in biocompatibility, expandability, elasticity, and flexibility. In addition, a particle diameter of embolization particles according to the exemplary embodiments of the present disclosure may vary from about 50 µm to about 2,000 pm, and the embolization particles according to the exemplary embodiments of the present disclosure exhibit a porous sphere of a uniform sponge structure. Accordingly, the embolization particles according to the exemplary embodiments of the present disclosure can minimize vascular stimulation.

In addition, the preparation of microparticles for the embolization by the method of preparing microparticles for embolization of the present disclosure is characterized by crosslinking the gelatin after selectively controlling the molecular weight of the gelatin, and dropping or spraying the gelatin on oil and fat, and thereby the gelatin in a gel state is prevented from being aggregated, porous spheres having a uniform sponge structure with a particle size of about 30 µm to about 4,000 pm, which can predict and control a time of intravascular embolism absorption, can be easily prepared.

The gelatin generally gels at a low temperature and has a characteristic that its viscosity decreases as the molecular weight decreases. In a case where an average molecular weight is about 10,000 or less, the gelatin does not gel at low temperature and remains in a liquid state, and there is a problem that a crosslinking process is difficult. Thus, the embolization particles having the average molecular weight of about 10,000 or less have never been developed. However, the embolization particles according to the exemplary embodiments of the present disclosure are granulated with the gelatin having a mixed molecular weight of about 10,000 to about 50,000 to develop embolization particles having an average molecular weight of about 5,000 to about 10,000. While the embolization particles do not stably dissolve in the blood at a body temperature level (about 37°C) for a certain period of time, low-molecular gelatin embolization particles are firstly degraded and absorbed in vivo by pepsin, which is a proteolytic enzyme secreted by macrophages in blood vessels, and high-molecular gelatin embolization particles are degraded and absorbed in vivo in stages, and thereby a vascular embolization time can be adjusted by adjusting the molecular weight and a ratio of the gelatin embolization particles.

### BRIEF DESCRIPTION OF THE DRAWINGS

FIG. 1 is a flow chart illustrating a process in which embolization particles are prepared according to exemplary embodiments of the present disclosure.

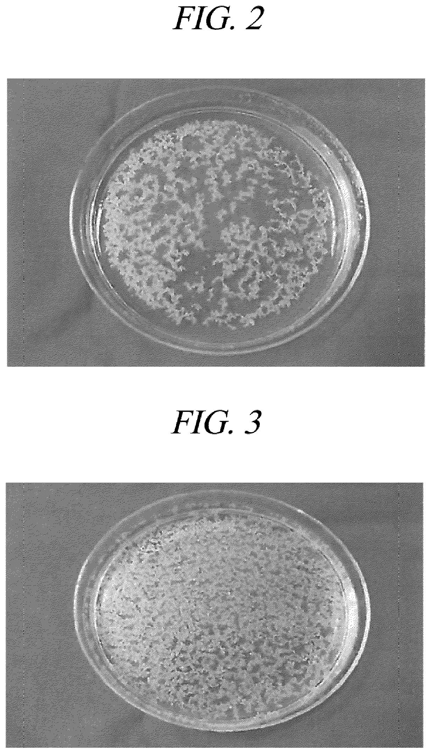

FIG. 2 is a photograph of embolization particles prepared according to Example 7 of the present disclosure.
FIG. 3 is a photograph of embolization particles prepared according to Example 9 of the present disclosure.

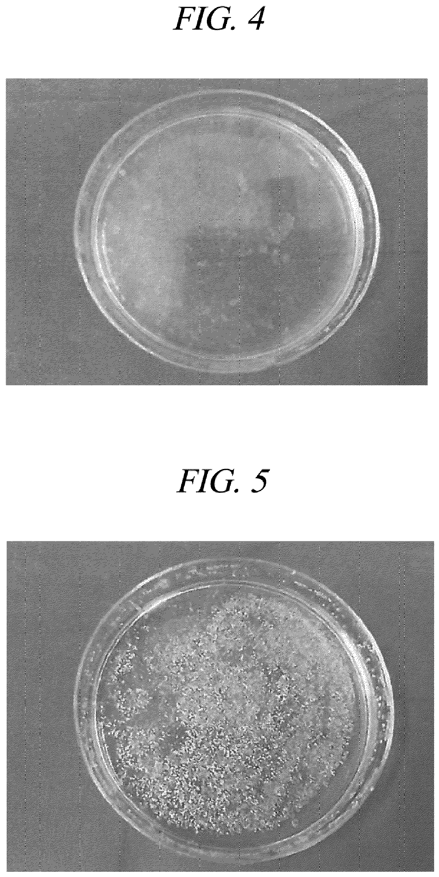

FIG. 4 is a photograph after the embolization particles prepared according to Example 7 of the present disclosure are left at room temperature for 10 days.
FIG. 5 is a photograph after the embolization particles prepared according to Example 9 of the present disclosure are left at room temperature for 10 days.
FIGS. 6A and 6B are angiographic photographs immediately before and immediately after administration of high-molecular weight gelatin vascular embolization microspheres of group A, respectively, and FIGS. 6C and 6D are angiographic photographs immediately before and immediately after administration of low-molecular weight gelatin vascular embolization microspheres of group B, respectively.
FIGS. 7A to 7C are angiographic photographs immediately before, immediately after, and 2 days after the administration of high-molecular weight gelatin vascular embolization microspheres of group C, respectively, and FIGS. 7D to 7F are angiographic photographs immediately before, immediately after, and 2 days after the administration of low-molecular weight gelatin vascular embolization microspheres of group D, respectively.
FIGS. 8A and 8B are photographs illustrating comparison of tissue sections and renal tissue occlusion 2 days after the administration of high-molecular weight gelatin vascular embolization microspheres and low-molecular weight gelatin vascular embolization microspheres, respectively.
FIGS. 9A to 9C are angiographic photographs immediately before, immediately after, and 3 weeks after the administration of high-molecular weight gelatin vascular embolization microspheres of group E, respectively, and FIGS. 9D to 9F are angiographic photographs immediately before, immediately after, and 3 weeks after the administration of low-molecular weight gelatin vascular embolization microspheres of group F, respectively.
FIGS. 10A and 10B are photographs illustrating renal vascular embolism recanalization and occlusion results through angiography and tissue sections 21 days after the administration of high-molecular weight gelatin vascular embolization microspheres and low-molecular weight gelatin vascular embolization microspheres, respectively.

### DETAILED DESCRIPTION

Hereinafter, exemplary embodiments and examples of the present disclosure will be described in detail with reference to the accompanying drawings so that the present disclosure may readily implemented by those skilled in the art. However, it is to be noted that the present disclosure may be implemented in various different forms and is not limited to the exemplary embodiments and examples described herein. In drawings, parts irrelevant to the description are omitted for the simplicity of explanation, and similar reference numerals are denoted to similar parts throughout the whole document.

Throughout the whole document, the term "connected to" or "coupled to" that is used to designate a connection or coupling of one element to another element includes both a case that an element is "directly connected or coupled to" another element and a case that an element is "electronically connected or coupled to" another element via still another element.

Throughout the whole document, the term "on" that is used to designate a position of one element with respect to another element includes both a case that the one element is adjacent to the other element and a case that any other element exists between these two elements.

Throughout the whole document, the term "comprises or includes" and/or "comprising or including" used in the document means that one or more other components, steps, operation and/or existence or addition of elements are not excluded in addition to the described components, steps, operation and/or elements unless context dictates otherwise.

As used herein, the term "about or approximately" or "substantially" is intended to have meanings close to numerical values or ranges specified with an allowable error and intended to prevent accurate or absolute numerical values disclosed for understanding of the present disclosure from being illegally or unfairly used by any unconscionable third party.

As used throughout the whole document, the term "step to" or "step of" does not mean "step for".

Throughout the whole document, the term "combination of" included in Markush type description means mixture or combination of one or more components, steps, operations and/or elements selected from a group consisting of components, steps, operation and/or elements described in Markush type and thereby means that the disclosure includes one or more components, steps, operations and/or elements selected from the Markush group.

Throughout the whole document, a phrase in the form "A and/or B" means "A or B, or A and B".

Throughout the whole document, the term "alkyl" or "alkyl group" includes linear or branched alkyl groups having 1 to 20 carbon atoms, 1 to 12 carbon atoms, 1 to 10 carbon atoms, 1 to 8 carbon atoms, or 1 to 5 carbon atoms, and all possible isomers thereof. For example, the alkyl or alkyl group includes methyl group (Me), ethyl group (Et), n-propyl group (ⁿPr), iso-propyl group (^{iso}Pr), n-butyl group (ⁿBu), iso-butyl group (^{iso}Bu) , tert-butyl group (tert-Bu, ^{t}Bu), sec-butyl group (sec-Bu, ^{sec}Bu), n-pentyl group (ⁿPe), iso-pentyl group (^{iso}Pe), sec-pentyl group (^{sec}Pe), tert-Pentyl group (^{t}Pe), neo-pentyl group (^{neo}Pe), 3-pentyl group, n-hexyl group, iso-hexyl group, heptyl group, 4,4-dimethylpentyl group, octyl group, 2,2,4-trimethylpentyl group, nonyl group, decyl group, undecyl group, dodecyl group, isomers thereof, and the like, but may not be limited thereto.

Throughout the whole document, the term "average molecular weight" may mean "weight average molecular weight".

Hereinafter, the exemplary embodiments of the present application are described in detail, but the present application may not be limited thereto.

A first aspect of the present disclosure provides embolization particles according to claim 1.

In one exemplary embodiment of the present disclosure, the polypeptide is derived from gelatin and can be obtained by hydrolyzing the gelatin. The gelatin may include mammal-derived gelatin or fish-derived gelatin, and in particular, in a case where the gelatin includes the fish-derived gelatin, it is possible to solve a concern of occurrence of mad cow disease or foot-and-mouth disease caused by gelatin of the related art using cows or pigs. The fish-derived gelatin is prepared from fish skin of warm-water fish species, and can be gelled at a lower temperature (for example, about 20 °C to about 40 °C) compared to the gelatin using cows or pigs.

In one exemplary embodiment of the present disclosure, embolization particles may have uniform porous spheres including micropores. For example, the embolization particles may have a sponge structure. In one exemplary embodiment of the present disclosure, the porosity of embolization particles is about 20 vol% to about 70 vol%, about 20 vol% to about 60 vol%, about 20 vol% to about 50 vol%, about 20 vol% to about 40 vol%, about 20 vol% to about 30 vol%, about 30 vol% to about 70 vol%, about 30 vol% to about 60 vol%, about 30 vol% to about 50 vol%, about 30 vol% to about 40 vol%, about 40 vol% to about 70 vol%, about 40 vol% to about 60 vol%, or about 40 vol% to about 50 vol%, but is not limited thereto.

In one exemplary embodiment of the present disclosure, the average particle diameter of embolization particles may be about 10 µm to about 5,000 pm, but is not limited thereto. For example, the particle diameter of the embolization particles is about 10 µm to about 5,000 pm, about 10 µm to about 4,500 pm, about 10 µm to about 4,000 µm, about 10 µm to about 3,000 pm, about 10 µm to about 2,000 pm, about 10 µm to about 1,000 pm, about 20 µm to about 5,000 pm, about 20 µm to about 4,500 pm, about 20 µm to about 4,000 pm, about 20 µm to about 3,000 pm, about 20 µm to about 2,000 pm, about 20 µm to about 1,000 pm, about 30 µm to about 5,000 pm, about 30 µm to about 4,500 pm, about 30 µm to about 4,000 pm, about 30 µm to about 3,000 pm, about 30 µm to about 2,000 pm, or about 30 µm to about 1,000 pm, but is not limited thereto.

In one exemplary embodiment of the present disclosure, the average molecular weight of embolization particles is about 5,000 to about 30,000, about 5,000 to about 25,000, about 5,000 to about 20,000, about 5,000 to about 15,000, about 5,000 to about 10,000, about 10,000 to about 30,000, about 10,000 to about 25,000, about 10,000 to about 20,000, about 10,000 to about 15,000, about 15,000 to about 30,000, about 15,000 to about 25,000, about 15,000 to about 20,000, about 20,000 to about 30,000, about 20,000 to about 25,000, or about 25,000 to about 30,000. In one exemplary embodiment of the present application, the average molecular weight of embolization particles may be about 5,000 to about 15,000. In one exemplary embodiment of the present disclosure, the average molecular weight of the embolization particles may be about 15,000 to about 30,000.

The embolization particle according to one exemplary embodiment of the present disclosure is formed by cross-linking gelatin having a relatively large average molecular weight and gelatin having a relatively small average molecular weight. Accordingly, the embolization particles according to one exemplary embodiment of the present disclosure may have a desired particle size and a desired degradation rate. For example, the average diameter of embolization particles according to one exemplary embodiment of the present disclosure may be determined by the content and average molecular weight of gelatin having a relatively large average molecular weight. In addition, the degradation rate of embolization particles according to one exemplary embodiment of the present disclosure may be determined by the content and average molecular weight of gelatin having a relatively small average molecular weight.

In one exemplary embodiment of the present disclosure, the embolization particles may include a first polypeptide having a first average molecular weight; and a second polypeptide having a second average molecular weight smaller than the first average molecular weight. The first polypeptide and the second polypeptide may be cross-linked with each other but are not limited thereto. Here, the first average molecular weight may be about 40,000 to about 100,000, about 15,000 to about 39,000, or about 5,000 to about 14,000, and the second average molecular weight may be about 15,000 to about 39,000, about 5,000 to about 14,000, or about 500 to about 4,000, but are not limited thereto. Alternatively, the first average molecular weight may be about 40,000 to about 60,000, about 15,000 to about 30,000, or about 7,000 to about 12,000, and the second average molecular weight may be about 15,000 to about 30,000, about 7,000 to about 12,000, or about 1,000 to about 2,000, but are not limited thereto.

In one exemplary embodiment of the present disclosure, based on 100 parts by weight of the total polypeptide, about 60 parts by weight to about 80 parts by weight of the first polypeptide, and about 20 parts by weight to about 40 parts by weight of the second polypeptide may be included, but are not limited thereto. In one exemplary embodiment of the present disclosure, based on 100 parts by weight of the total polypeptide, about 20 parts by weight to about 40 parts by weight of the first polypeptide, and about 60 parts by weight to about 80 parts by weight of the second polypeptide may be included, but are not limited thereto.

In one exemplary embodiment of the present disclosure, the embolization particles may further include a third polypeptide having a third average molecular weight less than the second average molecular weight, and the first polypeptide, the second polypeptide, and the third polypeptide may be cross-linked with each other, but may not be limited thereto. Here, the first average molecular weight is about 40,000 to about 100,000, or about 15,000 to about 39,000, the second average molecular weight is about 15,000 to about 39,000, or about 5,000 to about 14,000, and the third average molecular weight is about 5,000 to about 14,000, or about 500 to about 4,000, but are not limited thereto. Alternatively, the first average molecular weight is about 40,000 to about 60,000, or about 15,000 to about 30,000, the second average molecular weight is about 15,000 to about 30,000, or about 7,000 to about 12,000, and the third average molecular weight is about 7,000 to about 12,000, or about 1,000 to about 2,000, but are not limited thereto.

In one exemplary embodiment of the present disclosure, based on 100 parts by weight of the total polypeptide, about 60 parts by weight to about 80 parts by weight of the first polypeptide, about 10 parts by weight to about 30 parts by weight of the second polypeptide, and about 10 parts by weight to about 30 parts by weight of the third polypeptide may be included, but are not limited thereto.

In one exemplary embodiment of the present disclosure, the polypeptide may more contain the polypeptide having a relatively high average molecular weight than the polypeptide having a relatively low average molecular weight, in this case, the polypeptide formed by cross-linking embolization particles degrades relatively slowly in the body.

In one exemplary embodiment of the present disclosure, the polypeptide may more contain the polypeptide having a relatively low average molecular weight than the polypeptide having a relatively high average molecular weight, and in this case, the polypeptide formed by cross-linking embolization particles degrades relatively quickly in the body.

In one exemplary embodiment of the present disclosure, the polypeptide may include a carboxylic acid group and/or an amine group. That is, the first polypeptide, the second polypeptide, and the third polypeptide may include the carboxylic acid group and/or the amine group.

In one exemplary embodiment of the present disclosure, the polypeptides are the same as each other or are derived from different gelatins, and may include at least one amino acids selected from glycine, proline, hydroxyproline, glutamic acid, arginine, and alanine, but are not limited thereto.

In one exemplary embodiment of the present disclosure, the first polypeptide, the second polypeptide, and the third polypeptide may each include at least one of the following Chemical Formulas 1 to 6:

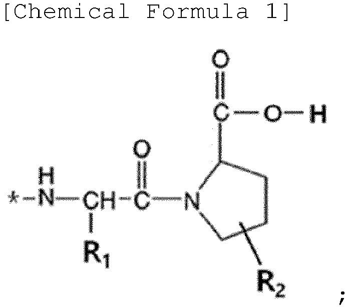

Here, R₁ is hydrogen or a substituted or unsubstituted C₁₋₂₀ alkyl group, and R₂ is hydrogen or an alcohol group.

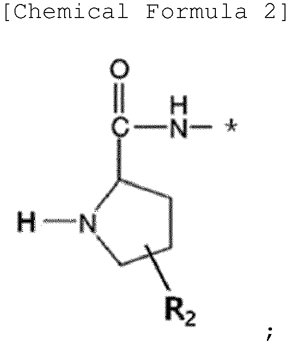

Here, R₂ is hydrogen or an alcohol group.

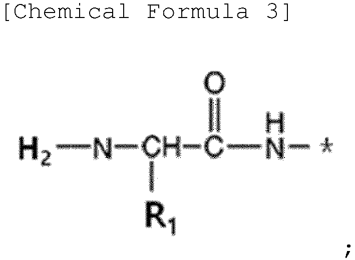

Here, R₁ is hydrogen or a substituted or unsubstituted C₁₋₂₀ alkyl group.

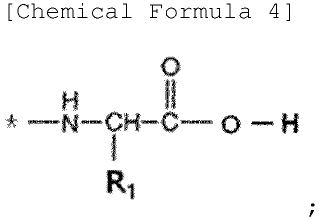

Here, R₁ is hydrogen or a substituted or unsubstituted C₁₋₂₀ alkyl group.

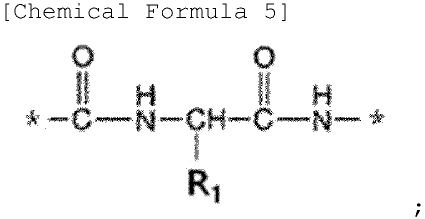

Here, R₁ is hydrogen or a substituted or unsubstituted C₁₋₂₀ alkyl group.

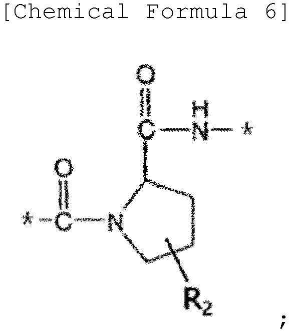

Here, R₂ is hydrogen or an alcohol group.

In addition, in Formulas 3 to 5, R1 may be represented by Formula 7 or Formula 8 below.

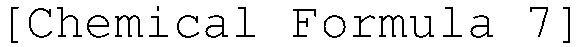

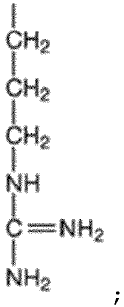

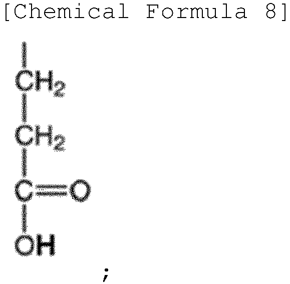

A second aspect of the present disclosure provides a method for preparing embolization particles according to claim 4.

Detailed descriptions on the second aspect of the present disclosure, which overlap with those on the first aspect of the present disclosure, are omitted hereinafter, but the descriptions of the first aspect of the present disclosure may be identically applied to the second aspect of the present disclosure, even though they are omitted hereinafter.

In one exemplary embodiment of the present disclosure, the polypeptide is derived from gelatin, and may be obtained by hydrolyzing the gelatin. The gelatin may include mammal-derived gelatin or fish-derived gelatin, and in particular, in a case where the gelatin includes the fish-derived gelatin, it is possible to solve the concern of occurrence of the mad cow disease or the foot-and-mouth disease caused by gelatin using of the related art cows or pigs. The fish-derived gelatin is prepared from fish skin of warm-water fish species and can be gelled at a lower temperature (for example, about 20°C to about 40°C) compared to gelatin using cows or pigs.

In one exemplary embodiment of the present disclosure, embolization particles may be formed at a relatively low temperature and may have uniform porous spheres.

In one exemplary embodiment of the present disclosure, the average molecular weight of embolization particles is about 30,000 or less, about 25,000 or less, about 20,000 or less, about 15,000 or less, about 5,000 to about 30,000, about 5,000 to about 25,000, about 5,000 to about 20,000, about 5,000 to about 15,000, about 5,000 to about 10,000, about 10,000 to about 30,000, about 10,000 to about 25,000, about 10,000 to about 20,000, about 10,000 to about 15,000, about 15,000 to about 30,000, about 15,000 to about 25,000, about 15,000 to about 20,000, about 20,000 to about 30,000, about 20,000 to about 25,000, or about 25,000 to about 30,000, but may not be limited thereto. In one exemplary embodiment of the present application, the average molecular weight of the embolization particles may be about 5,000 to about 15,000. In one exemplary embodiment of the present application, the average molecular weight of the embolization particles may be about 15,000 to about 30,000.

In one exemplary embodiment of the present disclosure, the hydrolysis may be performed by subcritical water hydrolysis.

In one exemplary embodiment of the present disclosure, the polypeptides may be cross-linked by using a cross-linking agent. The cross-linking agent may be at least one selected from formaldehyde, glutaraldehyde, dialdehyde starch, epoxy compound, glutaraldehyde glyoxal, glyoxal, 2,2-dimethoxy-2-phenyl acetophenone, tripolyphosphate sodium salt, diacetaldehyde PEG, scleraldehyde, diethylsquarate, epichlorohydrin, genipin, tannins, phenylpropanoids, catechins, resveratrol, flavonoids (quercetin or the like), and isoflavonoids (isoflavones or the like), but is not limited thereto.

In one exemplary embodiment of the present disclosure, based on 100 parts by weight of the polypeptide, the cross-linking agent may be included as about 0.25 parts by weight to about 50 parts by weight, but is not limited thereto. For example, based on 100 parts by weight of the polypeptide, the cross-linking agent may be included about 0.25 parts by weight to about 50 parts by weight, about 0.25 parts by weight to about 40 parts by weight, about 0.25 parts by weight to about 30 parts by weight, about 0.25 parts by weight to about 20 parts by weight, about 1 part by weight to about 50 parts by weight, about 1 part by weight to about 40 parts by weight, about 1 part by weight to about 30 parts by weight, about 1 part by weight to about 20 parts by weight, about 2 parts by weight to about 50 parts by weight, about 2 parts by weight to about 40 parts by weight, about 2 parts by weight to about 30 parts by weight, about 2 parts by weight to about 20 parts by weight, about 3 parts by weight to about 50 parts by weight, about 3 parts by weight to about 40 parts by weight, about 3 parts by weight to about 30 parts by weight, or about 3 parts by weight to about 20 parts by weight, but is not limited thereto. In one exemplary embodiment of the present application, based on 100 parts by weight of the polypeptide, the cross-linking agent may be included about 3 parts by weight to about 20 parts by weight.

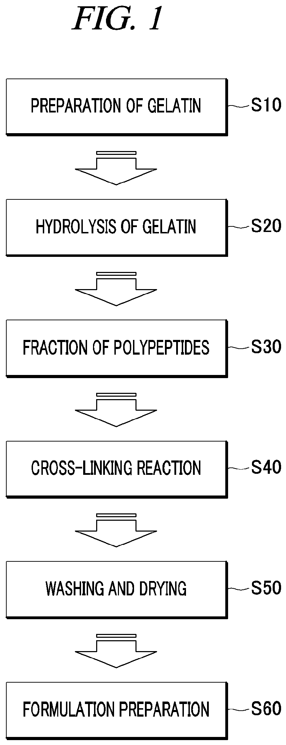

Referring to FIG. 1, it can be prepared by the following process according to an exemplary embodiment of the present disclosure:

First, gelatin is prepared (S10). The gelatin may be mammal-derived gelatin or fish-derived gelatin. The weight average molecular weight of the gelatin may be 50,000 to 100,000. The gelatin may be obtained from mammalian collagen or fish collagen. Alternatively, the gelatin is commercially available. For the gelatin, the above description may be referred to.

Thereafter, the gelatin is hydrolyzed (S20). The gelatin may be hydrolyzed in a subcritical water state. In more specifically, the gelatin is dissolved or dispersed in water to form an aqueous solution or an aqueous dispersion. At this time, the content of gelatin in the aqueous solution or dispersion may be about 3wt% to about 20wt%.

Thereafter, the aqueous solution or the aqueous dispersion is maintained in the subcritical water state for a certain period of time. For example, the aqueous solution or aqueous dispersion may be maintained for about 10 minutes to about 5 hours at a temperature of about 100°C to about 250°C, in a state of about 30 to about 80 atm. Accordingly, the gelatin is hydrolyzed and polypeptide is formed. In addition, the hydrolysis process in the subcritical water state may be proceeded in carbon dioxide (CO₂), nitrogen (N₂) atmosphere.

In addition, the gelatin can be hydrolyzed by enzymes. In addition, the gelatin can be hydrolyzed by acid. The gelatin can be hydrolyzed simultaneously by the subcritical water and the enzyme.

The polypeptide can be obtained by a single hydrolysis process, or by several hydrolysis processes to which various process conditions are applied. The polypeptide can be obtained by a single hydrolysis process from gelatin or can be obtained by several hydrolysis processes from different gelatins.

Then, the polypeptides are fractionated (S30). The polypeptides can be fractionated according to the molecular weight by gel filtration chromatography and using water as a solvent. The polypeptide is fractionated by HPLC (1260 HPLC system, Agilent, USA) to which a fraction collector (G1364C 1260 FC-AS, Agilent, USA) is connected, and can be divided according to the molecular weight. Polypeptides divided according to the molecular weight can be obtained by various methods capable of fractionating oligomer or polymer by molecular weight. The polypeptide can be separated by molecular weight through a column. Accordingly, the polypeptide according to the molecular weight can be obtained. For example, a first polypeptide, a second polypeptide, and a third polypeptide can be obtained.

As described above, the first polypeptide, the second polypeptide, and the third polypeptide have a first average molecular weight, a second average molecular weight, and a third average molecular weight, respectively. That is, the gelatin is hydrolyzed, and thus the obtained polypeptide is fractionated, so that the polypeptide having a desired average molecular weight can be prepared.

Thereafter, the polypeptide is cross-linked by the cross-linking agent (S40). More specifically, the polypeptides having desired weight average molecular weights are combined and can be cross-linked by the cross-linking agent. For example, at least two or more of the first polypeptide, the second polypeptide, and the third polypeptide may be combined and cross-linked by the cross-linking agent. For example, the first polypeptide and the second polypeptide may be cross-linked. The first polypeptide, the second polypeptide, and the third polypeptide may be cross-linked by the cross-linking agent.

In one exemplary embodiment of the present disclosure, the cross-linking reaction is carried out in an aqueous solution containing the polypeptide in a content of about 3wt% to about 10wt%. After the cross-linking agent is added to the aqueous solution in the content described above, the polypeptide is cross-linked by the cross-linking agent at a temperature of about 20°C to about 40°C for about 6 hours to about 12 hours. For example, the polypeptide aqueous solution and the cross-linking agent solution are mixed in a weight ratio of 100:0.5 to 100:10. Thereafter, the mixed solution is firstly stirred at a temperature of about 20°C to about 40°C, at about 1,000 rpm to about 1, 500 rpm, for about 5 minutes to about 120 minutes. Thereafter, the firstly stirred mixed solution is secondarily stirred at a temperature of about 20°C to about 40°C at about 1 rpm to about 500 rpm for about 10 minutes to about 48 hours. Accordingly, a gelatin aqueous solution in which the polypeptide is cross-linked can be prepared. More specifically, in the first stirring step, in a case where the stirring speed is out of the range of the stirring speed of about 1,000 rpm to about 1,500 rpm, bubbles are formed, and a problem of lowering the density may occur. In addition, in the first stirring step, in a case where the mixture is stirred at a temperature of 20°C or less, the mixed solution may be gelled. In addition, in the first stirring step, in a case where the mixed solution is stirred at a temperature of 40°C or higher, a problem of lowering the density may occur as bubbles are generated.

In addition, in the second stirring step, when the stirring speed exceeds 500 rpm, as the mixed solution is gelled, a problem that cannot be easily cross-linked in a sol state may occur, and water absorption is significantly lowered. Accordingly, in a case where the stirring speed exceeds 500 rpm, the functionality as microparticles for embolization may be deteriorated. In addition, in the second stirring step, in a case where the temperature is out of the range described above, the gelatin cross-linked aqueous solution may not be easily prepared as the mixed solution is gelled and hardened. In addition, the stirring time in the second stirring is 10 minutes-48 hours to improve absorption and maintain a uniform shape.

Accordingly, the gelatin aqueous solution cross-linked by the cross-linking process is prepared. Thereafter, the cross-linked gelatin aqueous solution was dropped and sprayed on the oil and fat, thereby preventing the gelatinous cross-linked gelatin from agglomeration, and preparing porous spherical cross-linked gelatin particles having a uniform sponge structure with a particle diameter of about 30 µm to 4,000 µm. In addition, since the degree to which the cross-linked gelatin is exposed to oil and fat is low, the oil and fat can be easily separated from the cross-linked gelatin.

The cross-linked gelatin particles having the spherical sponge structure are formed. The cross-linked gelatin particles may have various average particle diameters depending on the content of the polypeptide according to the average molecular weight, the content of the cross-linking agent, the cross-linking temperature, and the cross-linking time. For example, the cross-linked gelatin particles may have average particle diameters of about 30 µm to about 4,000 µm depending on the conditions described above.

More specifically, the gelatin may be fish gelatin. That is, the gelatin used for the hydrolysis may be derived from fish. In particular, the fish gelatin is prepared from the fish skin of warm-water fish species and may have a property of gelatinizing at a lower temperature than that of the gelatin using cows or pigs. Accordingly, the polypeptide derived from the fish gelatin is easily cross-linked in the sol state at a temperature of about 20°C to about 40°C, thereby forming the porous sphere having the uniform sponge structure with the particle size of about 30 µm to about 4,000 µm. In addition, when the polypeptide is derived from the fish gelatin, the viscosity of the non-gelatinized state is low, and the molecular weight is small, so that it may be dropped or sprayed at a higher concentration. Accordingly, in the present example, gelatin microparticles having a high visible density can be prepared.

In addition, the concentration of the polypeptide aqueous solution derived from the fish gelatin may be 3wt% to about 20wt%. Further, in the second stirring step, the concentration of the cross-linking agent in the prepared gelatin cross-linked aqueous solution may be about 0.05wt% to about 10wt%.

More specifically, the gelatin microparticle emulsion is sprayed or dropped into oil and fat through a nozzle by compressed air in an encapsulator. In this case, the temperature of the micorparticule solution may be about 20°C to about 40°C, and the temperature when passing through the nozzle may be about 65°C to about 75°C. In addition, according to the frequency of about 40 Hz to 6,000 Hz, it is possible to control the size of the dropped or sprayed spherical gelatin droplets.

In addition, the oil and fat may be ester-based oil and fat, hydrocarbon oil and fat, animal oil and fat, or plant oil and fat. For example, the oil and fat are olive oil, soybean oil, canola oil, grapeseed oil, soybean oil, palm oil, paraffin oil, α-bisabolol, stearyl glycyrrhetinate, salicylic acid, tocopheryl acetate, panthenol, glyceryl stearate, cetyl octanolate, isopropyl myristate, 2-ethylene isopelanate, di-cl2-13 alkyl malate, ceteatyl octanoate, butylene glycol dicaptylate/dicaptylate (butylene glycol dicaptylate/dicaprate), isonyl isostearate, isostearyl isostearate, cetyl octanoate, octyldodecyl myristate, cetyl esters, C10-30 cholesterol/lanosterol ester, hydrogenated castor oil, mono-glycerides, diglycerides, triglycerides, beeswax, canauba wax, suctosedistearate, PEG-8 beeswax, candelaria wax, mineral oil, squalene, squalane, monoglyceride, diglyceride, triglyceride, medium-chain glyceride, miglyol, or cremophor.

Preferably, the oil and fat may be paraffin oil, but is not limited thereto, and more preferably, the oil and fat are characterized in that 0 parts by weight to about 5 parts by weight of the cross-linking agent is mixed with respect to 100 parts by weight of the oil and fat, thereby making it easier for the gelatin particles to maintain their spherical shapes.

In addition, the oil and fat exist by being cooled at a temperature of 0°C to about 100°C so that the gelatin can easily form the spherical shape.

Accordingly, the cross-linked spherical gelatin particles can be prepared.

Thereafter, the cross-linked gelatin particles are washed and dried (S50). After the oil and fat containing the spherical gelatin particles are agitated, and through washing and drying processes, porous gelatin microparticles having the sponge structure may be formed.

The cross-linked spherical gelatin particles may be washed with an organic solvent.

The oil and fat containing the spherical gelatin microparticles prepared in step S40 are stirred at a temperature of about 0°C to about 10°C at about 400 rpm to about 1,400 rpm, left to stand, and washed with the organic solvent. Accordingly, the oil and fat, and cross-linking agents contained in the cross-linked spherical gelatin particles are removed, and the cross-linked spherical gelatin particles are solidified.

Thereafter, the solidified gelatin particles are lyophilized for about 3 hours to about 48 hours, and thus, the cross-linked porous gelatin microparticles having the sponge structure are formed. The sponge-structured porous gelatin microparticles formed at this time are characterized by having a particle size of about 30 µm to about 4,000 µm.

More specifically, in the washing and drying step, by stirring the oil and fat containing the gelatin microparticles at about 400 rpm to about 1,400 rpm, in addition to allowing the gelatin microparticles to be easily formed into the spherical shape, the spherical gelatin is made to have suitable physical properties.In addition, as the oil and fat are maintained in a temperature range of 0 to about 10°C, the spherical gelatin particles of the oil and fat are prevented from being agglomerated with each other to maintain the spherical shape.

In this case, the organic solvent described above is petroleum ether, ethyl ether, isopropyl acetate, n-propyl acetate, isobutyl acetate, n-butyl acetate, isobutyl isobutyrate, 2-ethylhexyl acetate, ethylene glycol diacetate C9 acetate, C10 acetate, methyl ethyl ketone, methyl isobutyl ketone, methyl isoamyl ketone, methyl (n-amyl) ketone, dibutyl ketone, cyclohexanone, isophorone, acetaldehyde, n-butylaldehyde, crotonaldehyde, 2-ethyl hexaldehyde, isobutylaldehyde, propionaldehyde, ethyl 3-ethoxypropionate, toluene, xylene, trichloroethane, propylene glycol monomethylethyl acetate, ethylene glycol monoethyl ether acetate, ethylene glycol monobutyl ether, diethylene glycol monobutyl ether acetate, dibutyl phthalate, idetyl phthalate, dimethyl phthalate, dioctyl phthalate, dioctyl terephthalate, butyl octylphthalate, butylbenzene phthalate, dioctyl adipate, triethylene glycol di-2-ethylhexanoate, trioctyl trimethylretate, glyceryl triacetate, glyceryl/tripropionine, 2,2,4-trimethyl-1,3-pentanediol diisobutylate, or a mixture thereof.

Preferably, the organic solvent is characterized in that it is ethyl ether or isopropyl acetate.

Hereinafter, the present disclosure will be described more specifically, using examples, but the following examples are only illustrative to aid understanding of the present disclosure, and the contents of the present disclosure are not limited to the following examples.

### [Examples]

### Preparation Example: Preparation of Polypeptide

About 10 g of fish gelatin (geltech, medical gelatin) with an average molecular weight of about 100,000 was prepared and added to about 200 mL of purified water. CO2 and N2 were added to the 5wt% gelatin solution, and a hydrolysis solution was prepared at a high temperature and high pressure of 110°C and 30 bar, in 30 minutes of a subcritical water state. The hydrolysis solution was fractionated by molecular weight by a gel permeation chromatography. Accordingly, a first polypeptide having a molecular weight of about 50,000, a second polypeptide having an average molecular weight of about 25,000, a third polypeptide having an average molecular weight of about 10,000, and a fourth polypeptide having an average molecular weight of about 1,500 were obtained.

### Example 1: Preparation of Cross-Linked Gelatin Particles

Thereafter, 1 g of each of the first polypeptide and the second polypeptide was dissolved in deionized water to prepare a polypeptide aqueous solution of about 5 wt%. Thereafter, 37 mL of formaldehyde was added to 100 mL of an aqueous solution containing 15 wt% methanol to prepare an aqueous cross-linking agent.

Thereafter, 0.2 mL of the cross-linking agent aqueous solution was added to 20 mL of the gelatin aqueous solution, and the polypeptide aqueous solution was first stirred for 30 minutes at a speed of 1,000 rpm at about 30°C, and was stirred a second time for 24 hours 24 at a speed of 400 rpm at 30°C
Accordingly, a gelatin aqueous solution cross-linked in a sol state was prepared.

Thereafter, the prepared gelatin cross-linked aqueous solution was injected into the Encapsulator B-390 (BUCHI, Switzerland) adjusted to a frequency of 400 Hz, an electronegativity of 1,200 V, and a nozzle temperature of 75°C, and the cross-linking agent of the Encapsulator B-By dropping into 100 mL of paraffin oil containing the cross-linking agent of the Encapsulator 390 is dropped, thereby forming spherical gelatin particles in the paraffin oil. At this time, the paraffin oil was assumed to contain 1 mL of 37% formaldehyde in 100 mL of paraffin oil.

Thereafter, the paraffin oil including the spherical gelatin particles was stirred at 0°C at 1,000 rpm, and then allowed to stand for 30 minutes.

Next, the spherical gelatin droplets were washed by adding isopropyl ethyl alcohol to solidify the spherical gelatin and remove paraffin oil and formaldehyde.

By lyophilizing the solidified gelatin spheres for 24 hours, the gelatin microparticles having a particle diameter of about 355 µm to about 500 µm were prepared in which the first polypeptide having a molecular weight of 50,000 and the second polypeptide having a molecular weight of 25,000 were formed by cross-linking.

Finally, the gelatin microparticles were prepared by mixing alone, in a 5:5 ratio.

### Examples 2-6 and Comparative Examples 1-4

Except that the contents of the first polypeptide, the second polypeptide, the third polypeptide, and the fourth polypeptide are illustrated in Table 1, for the rest, the cross-linked gelatin particles were prepared in the same manner as that in Example 1.

**Table 1**

| item | Contents of polypeptide according to molecular weight (g) | | | | Polypeptide aqueous solution (mL) | Cross-linking agent aqueous solution (mL) |
|---|---|---|---|---|---|---|
| | 50,000 | 25,000 | 10,000 | 1,500 | | |
| Example 1 | 1 | 1 | - | - | 20 mL | 0.2 mL |
| Example 2 | 1.5 | - | - | 0.5 | 20 mL | 0.2 mL |
| Example 3 | 1 | - | - | 1 | 20 mL | 0.2 mL |
| Example 4 | 0.5 | - | - | 1.5 | 20 mL | 0.2 mL |
| Example 5 | - | 1.5 | 0.5 | - | 20 mL | 0.2 mL |
| Example 6 | - | 1 | 1 | - | 20 mL | 0.2 mL |
| Example 7 | - | 0.5 | 1.5 | - | 20 mL | 0.2 mL |
| Example 8 | 2 | - | - | - | 20 mL | 0.2 mL |
| Example 9 | - | 2 | - | - | 20 mL | 0.2 mL |
| Example 10 | 1 | - | 1 | - | 20 mL | 0.2 mL |
| Example 11 | - | 1 | - | 1 | 20 mL | 0.2 mL |

As illustrated in Table 2 below, the embolization particles according to the exemplary embodiments may have an improved embolic effect and a desired degradation period.

**Table 2**

| item | Particle diameter range (pm) | Presence or absence of embolic effect | degradation period |
|---|---|---|---|
| Example 1 | 355-500 | o | 30 days or more |
| Example 2 | 355-500 | ∘ | Within 15 days |
| Example 3 | 150-500 | ∘ | 5 days |
| Example 4 | 150-355 | ∘ | 5 days |
| Example 5 | 355-500 | ∘ | 4 days |
| Example 6 | 150-500 | ∘ | 4 days |
| Example 7 | 150-355 | ∘ | 2 days |
| Example 8 | 355-500 | ∘ | 30 days or more |
| Example 9 | 150-500 | ∘ | 30 days or more |
| Example 10 | 150-500 | ∘ | Within 20 days |
| Example 11 | 150-355 | ∘ | Within 15 days |

### Experimental Examples

### 1. Particle Size Analysis

The gelatin microparticles prepared in the Example described above were analyzed through a scanning electron microscope, and it was confirmed that gelatin microparticles having a uniform sponge structure with a particle diameter of about 150 µm to about 355 µm were prepared.

As described above, the present invention relates to the embolization microparticles and the method for preparing the same, and more specifically, by using the fish gelatins with average molecular weights of 50,000, 25,000, 10,000, and 1,500, embolization microparticles are prepared, which are porous spheres with the uniform sponge structure having the particle size of about 30 µm to about 4,000 pm, and thereby the embolization microparticles have excellent biocompatible, expandability, elasticity, and flexibility. Therefore, a vascular occlusion time can be easily predicted while minimizing vascular stimulation.

### 2. Dissolution Test Evaluation

In-vitro cell line and the embolization particles of the examples were mixed and left at room temperature. Thereafter, the embolization particles were observed with the naked eye to confirm whether they were degraded within a few days. Here, the "high-molecular gelatin vascular embolization microspheres" are gel-form particles prepared by cross-linking polypeptides having an average molecular weight of about 15,000 to about 30,000, and the "low-molecular gelatin vascular embolization microspheres" are gel-foam particles prepared by cross-linking polypeptides having an average molecular weight of about 5,000 to about 15,000.

Specifically, 100mg/12ea of the high-molecular gelatin vascular embolization microspheres and 100mg/12ea of the low-molecular gelatin vascular embolization microspheres were mixed with 20cc of saline solution (37°C), respectively, and a time to maintain the spherical shape was observed under a microscope.

As a result of observation, in the low-molecular gelatin vascular embolization microspheres (Low MW GMP), the spherical particles gradually disappear and disappear completely after 6 days, and in the high-molecular gelatin vascular embolization microspheres (High MW GMP), the spherical particles do not completely disappear even after 2 weeks. Therefore, it was confirmed that the low-molecular gelatin vascular embolization microspheres were absorbed more rapidly from the occurrence of partial decreasing than the high-molecular weight gelatin vascular embolization microspheres. Therefore, it is predicted that the low-molecular gelatin vascular embolization microspheres are rapidly absorbed even in blood vessels.

### 3. Animal Test Results

### (1) Test Summary

This experiment was conducted after approval by the Animal Experimental Ethics Committee (IACUC) of Yangsan Busan National University Hospital (PNUYH-2019-068), and a total of 26 New Zealand rabbits weighing 2.5 to 3.5Kg were used. Two of them were eliminated during the experiment (1 died during anesthesia and 1 failed ear artery puncture), and thereby the final 24 rabbits were experimented.

### (2) Test Procedure

The rabbit was fixed in the supine position under anesthesia, the central ear artery of the rabbit was punctured using 18G Medicut, and a 1.9F microcatheter was inserted into the central ear artery, thereby accessing to the rabbit arterial system. The renal artery was accessed through the aorta under fluoroscopy (Integris H5000F, Philips, Bothell, WA, USA), and renal artery angiography was performed using a contrast medium. After placing a microcatheter in the kidney lower pole artery, 0.1g of the high-molecular weight (prepared by cross-linking polypeptides of 15,000 to 30,000 Dalton) or the low-molecular weight (prepared by cross-linking polypeptides of 5,000 to 15,000 Dalton) gelatin vascular embolic microspherical particles were mixed with 10 mL of the contrast agent, 1 mL thereof was injected, the blood vessels were embolized, and then angiographic images were obtained. Thereafter, the rabbits were survived according to a schedule for each group, and after angiography was confirmed, the kidneys were removed and the pathological findings were analyzed.

### (3) Test Results

### 1) Measurement of Embolic Effect Immediately After Administration of High-Molecular and Low-Molecular Gelatin Vascular Embolization Microspheres

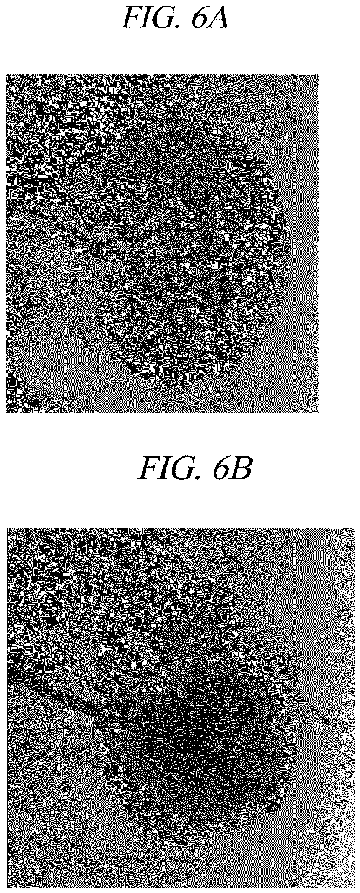

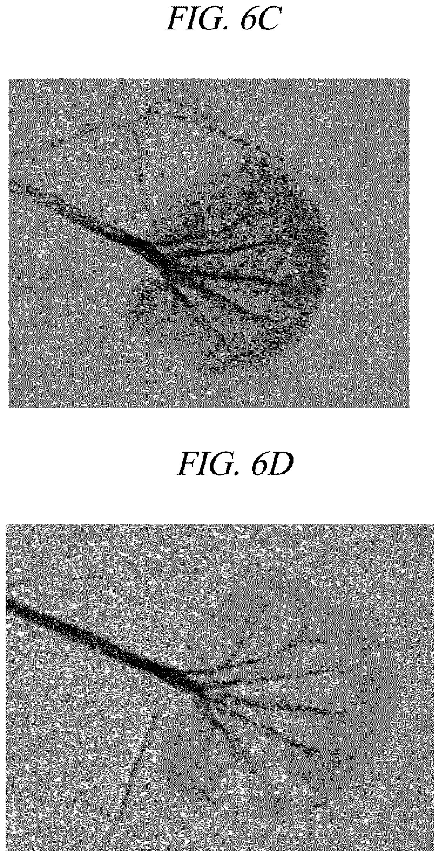

The high-molecular gelatin vascular embolization microspheres (group A, FIGS. 6A and 6B) and the low-molecular gelatin vascular embolization microspheres (Group B, FIGS. 6C and 6D) were administered to measure the embolic effect before and after the administration. As a result of confirming a blood flow by angiography before and after the surgical procedure, effective embolism was found in both groups A and B, and it was confirmed that there was no blood flow due to vascular occlusion in the renal vessels. In particular, it had a regular spherical form compared to the irregular gelatin vascular embolization microspheres of the related art, and was embolized in distal microvessels. In particular, group B had a lower viscosity during the surgical procedure than that of group A, so that the distal microvessels were selectively embolized in the microspherical form through microconductors.

### 2) Measurement of Embolic Effect 2 days after the administration of High-Molecular Weight and Low-Molecular Weight Gelatin Vascular Embolization Microspheres

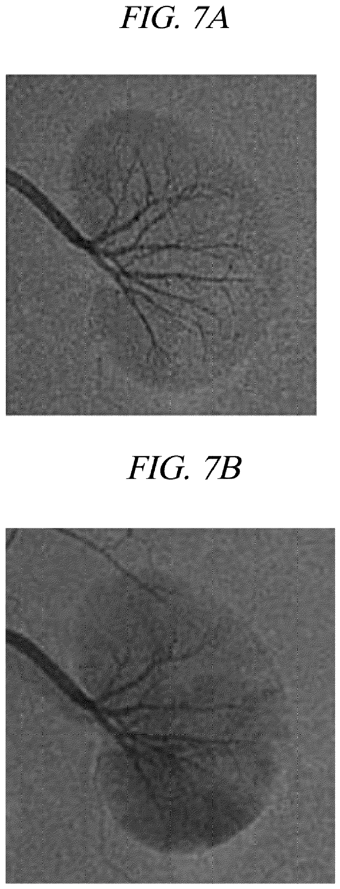

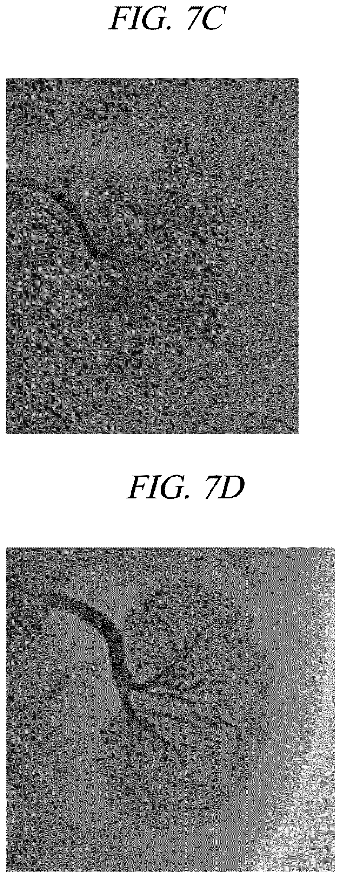

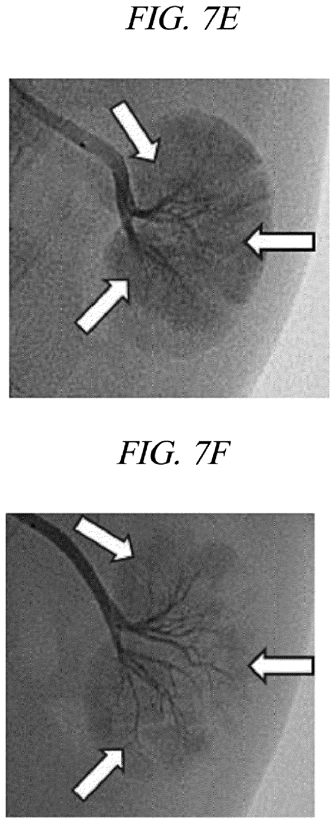

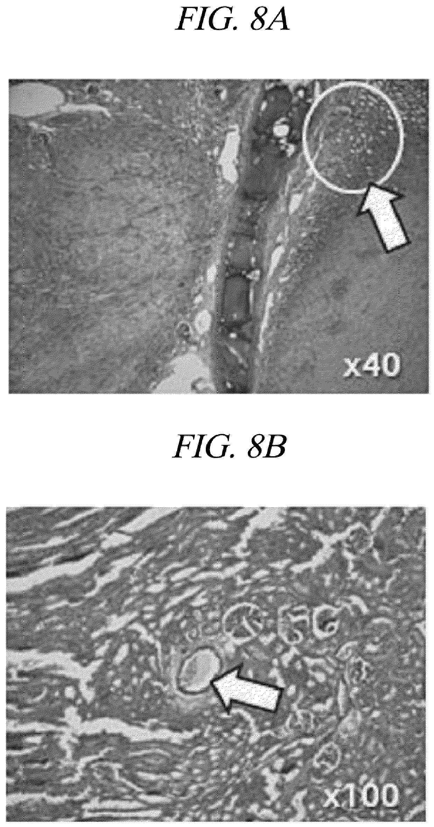

The high-molecular gelatin vascular embolization microspheres (group C, FIGS. 7A to 7C) and the low-molecular gelatin vascular embolization microspheres (Group D, FIGS. 7D to 7F) were administered to measure the embolic effect 2 days after the administration. As a result of confirming the flow of the blood by angiography before and after the surgical procedure, effective embolism was found on the angiographic images 2 days after the administration in the both groups C and D. In addition, as a result of comparing tissue sections and confirming renal tissue occlusion, group C showed an increase in vascular occlusion after 2 days the administration of the high-molecular gelatin vascular embolization microspheres, and gray-white (arrow) of the vascular occlusion site was observed (FIG. 8A), whereas the group D showed most absorption into blood vessels 2 days after the administration of the low-molecular gelatin vascular embolism microparticles, and the kidney was similar to normal tissues, and vascular occlusion was also recovered, thereby confirming that the chromaticity is similar to the surrounding normal vascular tissues (arrow) (FIG. 8B).

### 3) Measurement of Embolic effect After 21 Days Administration of High-Molecular Weight and Low-Molecular Weight Gelatin Vascular Embolization Microspheres

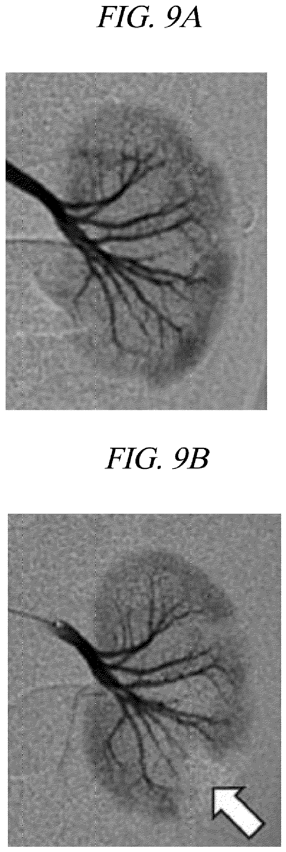

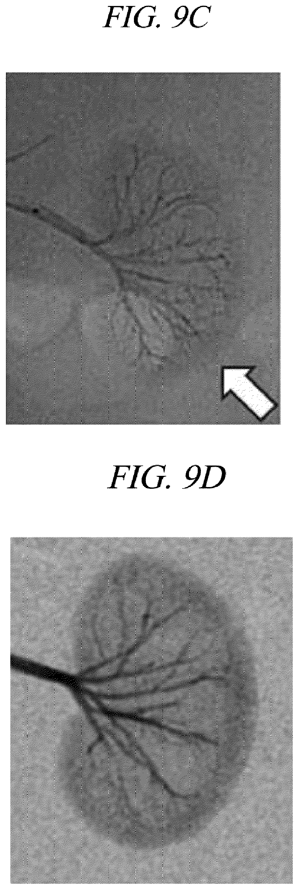

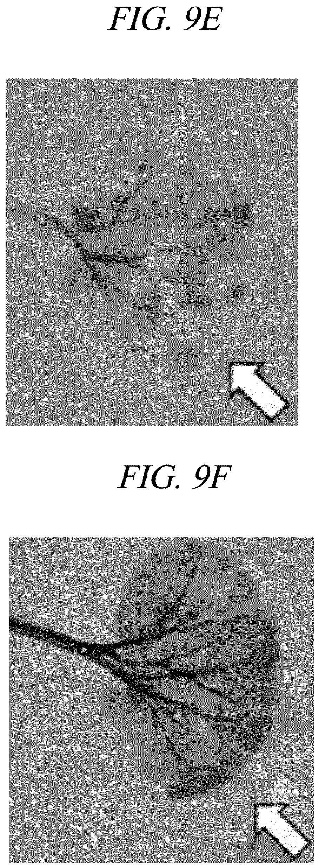

The high-molecular gelatin vascular embolization microspheres (group E, FIGS. 9A to 9C) and the low-molecular gelatin vascular embolization microspheres (Group F, FIGS. 9D to 9F) were administered to measure the embolic effect after 21 days the administration. As a result of confirming the flow of the blood by angiography before and after the surgical procedure, it was found that the renal blood flow in group F was recovered in a much wider range than that in group E. In particular, group E showed complete occlusion (vascular sclerosis) of distal microvascular 3 weeks after the administration, thereby indicating that the function of blood supply was lost, whereas in group F, when comparing tissue sections 3 weeks after the administration, distal microvascular was fully opened and the normal function of blood supply was restored.

### 4) Test Results

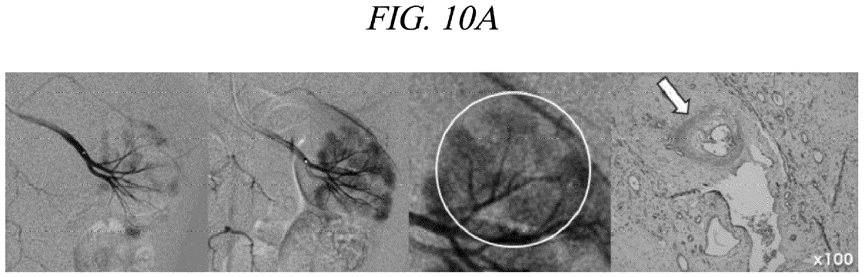

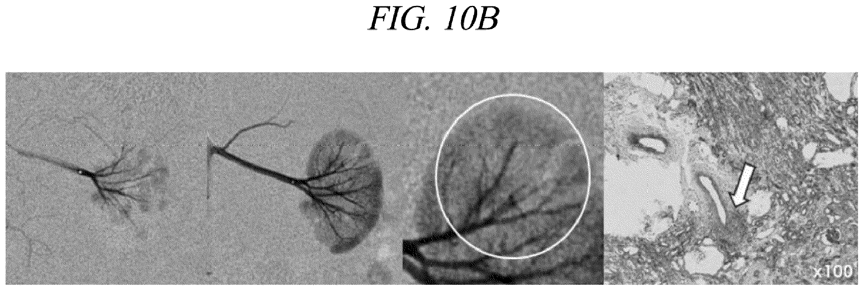

Both the high-molecular gelatin vascular embolization microspheres and the low-molecular gelatin vascular embolization microspheres showed effective embolic effect. Both groups had only partially reopened blood vessels to a similar degree 2 days after the administration, but in the angiography 3 weeks after the administration, it was confirmed that the low-molecular gelatin vascular embolization microspheres group recovered an amount of the renal blood flow in a much wider range than that in the high-molecular gelatin vascular embolization microspheres group. In addition, when comparing the recanalized blood vessels, the blood vessel walls were homogeneous in the group administered with low-molecular gelatin vascular embolization microspheres (FIG. 10B), whereas the blood vessel were narrowed in several places, and the inner diameters thereof were relatively irregular in the group administered with high-molecular gelatin vascular embolization microspheres (FIG. 10A). In pathologic examination, in the group administered with high-molecular gelatin vascular embolization microspheres, fibrosis progressed due to severe inflammation in the blood vessel wall and its surroundings, and a significant portion of the kidney was irreversibly fibrotic (FIG. 10A), whereas in the group administered with low-molecular gelatin vascular embolization microspheres, the blood vessel walls were normal, inflammation progressed only in a portion, and irreversible change in renal parenchyma was observed only in a much narrower range of the kidney (FIG. 10B).

### (4) Conclusions

The low molecular (prepared by cross-linking polypeptides of 5,000 to 15,000 Da) gelatin vascular embolization microspheres are superior to the high molecular (prepared by cross-linking polypeptides of 15,000 to 30,000 Da) gelatin vascular embolization microspheres in the selective embolization function of the original microvessel, and the embolism maintenance ability was 2 to 3 days, showing an effect on the restoration of normal function of the vascular embolization.

Specifically, both the high molecularic gelatin vascular embolization microspheres and the low-molecular weight gelatin vascular embolization microspheres exhibited effective vascular embolic effect as a whole, and low-molecular gelatin vascular embolization microspheres showed selective embolic effect on distal microvessels compared to high-molecular gelatin vascular embolization microspheres. When confirming the tissue section examination, low-molecular gelatin vascular embolization microspheres exhibited effective vascular embolic effect in 2 to 3 days, whereas high-molecular gelatin vascular embolization microspheres exhibited the embolic effect over 21 days. In particular, the low-molecular gelatin vascular embolization microspheres exhibited the vascular embolic effect only for 2 to 3 days, thereby restoring the normal function of blood flow supply to the embolized blood vessel portion, whereas the high-molecular weight gelatin vascular embolization microspheres exhibited the embolic effect for 21 days or more, and thereby the normal function of blood flow supply to the embolized blood vessel area was lost and the phenomenon of occlusion appeared, whereas the normal function of blood flow supply to the damaged vascular area was lost and the phenomenon of occlusion appeared.

The above description of the example embodiments is provided for the purpose of illustration, and it would be understood by those skilled in the art that various changes and modifications may be made without changing technical conception and essential features of the example embodiments. Thus, it is clear that the above-described example embodiments are illustrative in all aspects and do not limit the present disclosure. For example, each component described to be distributed can be implemented in a combined manner.

The scope of the inventive concept is defined by the following claims.

## Claims

1. Embolization particles, comprising:
at least a first polypeptide having a first average molecular weight; and
a second polypeptide having a second average molecular weight smaller than the first average molecular weight,
wherein the first average molecular weight is 40,000 to 100,000, 15,000 to 39,000, or 5,000 to 14,000, and
wherein the second average molecular weight is 15,000 to 39,000, 5,000 to 14,000, or 500 to 4,000;
wherein the first polypeptide and the second polypeptide are cross-linked with each other, and
wherein an average molecular weight of the embolization particles is 5,000 to 30,000.

2. The embolization particles of claim 1, further comprising:
a third polypeptide having a third average molecular weight smaller than the second average molecular weight,
wherein the first polypeptide, the second polypeptide, and the third polypeptide are cross-linked to each other,
wherein the first average molecular weight is 40,000 to 100,000, or 15,000 to 39,000,
wherein the second average molecular weight is 15,000 to 39,000, or 5,000 to 14,000, and
wherein the third average molecular weight is 5,000 to 14,000, or 500 to 4,000.

3. The embolization particles of claim 1,
wherein the polypeptides are derived from same gelatin or derived from different gelatins, and
wherein the polypeptides contain at least one amino acids selected from glycine, proline, hydroxyproline, glutamic acid, arginine, and alanine.

4. A method for preparing embolization particles, comprising:
cross-linking at least a first polypeptide having a first average molecular weight; and
a second polypeptide having a second average molecular weight smaller than the first average molecular weight, which are obtained by hydrolyzing gelatin, to obtain embolization particles;
wherein the first average molecular weight is 40,000 to 100,000, 15,000 to 39,000, or 5,000 to 14,000, and
wherein the second average molecular weight is 15,000 to 39,000, 5,000 to 14,000, or 500 to 4,000; and
wherein an average molecular weight of the embolization particles is 5,000 to 30,000.

5. The method for preparing embolization particles of claim 4,
wherein the hydrolysis is performed by subcritical water hydrolysis.

6. The method for preparing embolization particles of claim 4,
wherein the polypeptides are cross-linked by using a cross-linking agent, and
wherein the cross-linking agent is at least one selected from formaldehyde, glutaraldehyde, dialdehyde starch, epoxy compound, glutaraldehyde glyoxal, glyoxal, 2,2-dimetoxy-2-phenylacetophenone, tripolyphosphate sodium salt, diacetaldehyde PEG, scleraldehyde, diethylsquarate, epichlorohydrin genipine, tannins, phenylpropanoids, catechins, resveratrol, flavonoids, and isoflavonoids.

## Patentansprüche

1. Embolisationspartikel, welche umfassen:
mindestens ein erstes Polypeptid, das ein erstes mittleres Molekulargewicht aufweist; und
ein zweites Polypeptid, das ein zweites mittleres Molekulargewicht aufweist, welches kleiner ist als das erste Molekulargewicht,
wobei das erste mittlere Molekulargewicht 40000 bis 100000, 15000 bis 39000 oder 5000 bis 14000 beträgt, und
wobei das zweite mittlere Molekulargewicht 15000 bis 39000, 5000 bis 14000 oder 500 bis 4000 beträgt;
wobei das erste Polypeptid und das zweite Polypeptid miteinander vernetzt sind, und
wobei ein mittleres Molekulargewicht der Embolisationspartikel 5000 bis 30000 beträgt.

2. Embolisationspartikel nach Anspruch 1, welche zudem umfassen:
ein drittes Polypeptid, das ein drittes mittleres Molekulargewicht aufweist, welches kleiner ist als das zweite mittlere Molekulargewicht,
wobei das erste Polypeptid, das zweite Polypeptid und das dritte Polypeptid miteinander vernetzt sind,
wobei das erste mittlere Molekulargewicht 40000 bis 100000 oder 15000 bis 39000 beträgt,
wobei das zweite mittlere Molekulargewicht 15000 bis 39000 oder 5000 bis 14000 beträgt, und
wobei das dritte mittlere Molekulargewicht 5000 bis 14000 oder 500 bis 4000 beträgt.

3. Embolisationspartikel nach Anspruch 1,
wobei die Polypeptide von dem gleichen Gelatin oder von verschiedenen Gelatinen abgeleitet sind, und
wobei die Polypeptide mindestens eine Aminosäure enthalten, die aus Glycin, Prolin, Hydroxyprolin, Glutaminsäure, Arginin und Alanin ausgewählt ist.

4. Verfahren zur Herstellung von Embolisationspartikeln, welches umfasst:
Vernetzen mindestens eines ersten Polypeptids, das ein erstes mittleres Molekulargewicht aufweist; und
eines zweiten Polypeptids, das ein zweites mittleres Molekulargewicht aufweist, welches kleiner ist als das erste mittlere Molekulargewicht, die durch Hydrolysieren von Gelatin erhalten sind, um Embolisationspartikel zu erhalten;
wobei das erste mittlere Molekulargewicht 40000 bis 100000, 15000 bis 39000 oder 5000 bis 14000 beträgt, und
wobei das zweite mittlere Molekulargewicht 15000 bis 39000, 5000 bis 14000 oder 500 bis 4000 beträgt; und
wobei ein mittleres Molekulargewicht der Embolisationspartikel 5000 bis 30000 beträgt.

5. Verfahren zur Herstellung von Embolisationspartikeln nach Anspruch 4,
wobei die Hydrolyse durch Hydrolyse mit unterkritischem Wasser durchgeführt wird.

6. Verfahren zur Herstellung von Embolisationspartikeln nach Anspruch 4,
wobei die Polypeptide unter Verwendung eines Vernetzungsmittels vernetzt werden, und
wobei das Vernetzungsmittel mindestens eines ist, welches ausgewählt ist aus Formaldehyd, Glutaraldehyd, Dialdehydstärke, einer Epoxyverbindung, Glutaraldehyd-Glyoxal, Glyoxal, 2,2-Dimethoxy-2-phenylacetophenon, Tripolyphosphat-Natriumsalz, Diacetaldehyd-PEG, Scleraldehyd, Diethylquadratat, Epichlorhydrin-Genipin, Tanninen, Phenylpropanoiden, Catechinen, Resveratrol, Flavonoiden und Isoflavonoiden.

## Revendications

1. Particules d'embolisation, comprenant :
au moins un premier polypeptide ayant un premier poids moléculaire moyen ; et
un second polypeptide ayant un poids moléculaire moyen plus petit que le premier poids moléculaire moyen,
le premier poids moléculaire moyen étant de 40 000 à 100 000, de 15 000 à 39 000 ou de 5 000 à 14 000 et
le second poids moléculaire moyen étant de 15 000 à 39 000, de 5 000 à 14 000 ou de 500 à 4 000 ;
le premier polypeptide et le second polypeptide étant réticulés l'un avec l'autre et
un poids moléculaire moyen des particules d'embolisation étant de 5 000 à 30 000.

2. Particules d'embolisation selon la revendication 1, comprenant en outre :
un troisième polypeptide ayant un troisième poids moléculaire moyen plus petit que le deuxième poids moléculaire moyen,
le premier polypeptide, le deuxième polypeptide et le troisième polypeptide étant réticulés les uns aux autres,
le premier poids moléculaire moyen étant de 40 000 à 100 000 ou de 15 000 à 39 000,
le deuxième poids moléculaire moyen étant de 15 000 à 39 000 ou de 5 000 à 14 000 et
le troisième poids moléculaire moyen étant de 5 000 à 14 000 ou de 500 à 4 000.

3. Particules d'embolisation selon la revendication 1,
dans lesquelles les polypeptides sont dérivés de la même gélatine ou dérivées de gélatines différentes et
les polypeptides contenant au moins un acide aminé choisi parmi la glycine, la proline, l'hydroxyproline, l'acide glutamique, l'arginine, et l'alanine.

4. Procédé de préparation de particules d'embolisation, comprenant :
la réticulation d'au moins un premier polypeptide ayant un premier poids moléculaire moyen ; et
un second polypeptide ayant un second poids moléculaire moyen plus petit que le premier poids moléculaire moyen, qui sont obtenus par hydrolyse de la gélatine, pour obtenir des particules d'embolisation ;
le premier poids moléculaire moyen étant de 40 000 à 100 000, de 15 000 à 39 000 ou de 5 000 à 14 000 et
le second poids moléculaire moyen étant de 15 000 à 39 000, de 5 000 à 14 000 ou de 500 à 4 000 ; et
un poids moléculaire moyen des particules d'embolisation étant de 5 000 à 30 000.

5. Procédé de préparation de particules d'embolisation selon la revendication 4,
dans lequel l'hydrolyse est effectuée par hydrolyse d'eau sous-critique.

6. Procédé de préparation e particules d'embolisation selon la revendication 4, dans lequel les polypeptides sont réticulés en utilisant un agent de réticulation et
l'agent de réticulation étant au moins un choisi parmi le formaldéhyde, le glutaraldéhyde, l'amidon de dialdéhyde, un composé époxy, le glutaladé-hyde glyoxal, le glyoxal, la 2,2-méthoxy-2-phénylacétophénone, le sel de sodium de tripolyphosphate, le diacétaldéhyde PEG, le scléraldéhyde, le diéthylsquarate, l'épichlorhydrine génipine, des tanins, des phénylpropa-noïdes, des catéchines, le resvératrol, des falvonoïdes, et des isoflavo-noïdes.
